# EUROPEAN PATENT SPECIFICATION

(11) **EP 1 092 039 B1**
(45) Date of publication and mention of the grant of the patent: **29.02.2012**
(21) Application number: 99932081.5
(22) Date of filing: 29.06.1999
(51) Int. Cl.: C12P 21/06, C12P 19/34, C12N 15/74, C12Q 1/68

(54) **METHODS FOR GENERATING HIGHLY DIVERSE LIBRARIES**
VERFAHREN ZUR ERZEUGUNG VON HOCHGRADIG DIVERSEN BIBLIOTHEKEN
PROCEDES SERVANT A GENERER DES BANQUES EXTREMEMENT DIVERSIFIEES

(30) Priority: 29.06.1998 US 90970 P
(43) Date of publication of application: 18.04.2001
(73) Proprietor: Bristol-Myers Squibb Company, Princeton, NJ 08543-4000 (US)
(72) Inventor: WAGNER, Richard, Concord, MA 01742 (US); WRIGHT, Martin, C., Cambridge, MA 02139 (US); KREIDER, Brent, Bedford, MA 01730 (US)
(74) Representative: Reitstötter - Kinzebach
(86) International application number: PCT/US1999/014776
(87) International publication number: WO 2000/000632

(56) References cited:
- WO-A-97/20078
- WO-A-97/21837
- WO-A-98/41653
- WO-A-98/42728
- US-A- 4 994 379
- US-A- 5 447 839
- US-A- 5 580 730
- JOYCE G F ET AL: "A NOVEL TECHNIQUE FOR THE RAPID PREPARATION OF MUTANT RNAS" NUCLEIC ACIDS RESEARCH, OXFORD UNIVERSITY PRESS, SURREY, GB, vol. 17, no. 2, 25 January 1989 (1989-01-25), pages 711-722, XP000007446 ISSN: 0305-1048
- ALBERTS et al., "Recombinant DNA Technology", In: MOLECULAR BIOLOGY OF CELL, NEW YORK, GARLAND PUBLISHING, INC., 1994, pages 291-312, XP002924956.
- MATHEWS et al., BIOCHEMISTRY, REDWOOD CITY, THE BENJAMIN/CUMMINGS PUBLISHING COMPANY, 1990, pages 826, 827, 122-127 and 902-909, XP002924957.
- KACZOROWSKI et al., "Co-0perativity of Hexamer Ligation", GENE, 1996, Vol. 179, No. 1, pages 189-193, XP002924958.
- MINTZ et al., "EHD1-An EH-Domain-Containing Protein with a Specific Expression Pattern", GENOMICS, 01 July 1999, Vol. 59, No. 1, pages 66-76, XP002924959.

## Description

### Background of the Invention

In general, this invention relates to methods for generating and altering recombinant libraries.

The ability to isolate a desired nucleic acid or amino acid sequence requires the availability of recombinant libraries of sufficient number and diversity that a particular species is represented in the library and can be identified by one or more screening techniques. Such libraries facilitate the isolation of useful compounds, including therapeutics, research diagnostics, and agricultural reagents, as well as their coding sequences.

Moreover, desirable libraries may be specifically designed to contain large numbers of possible variants of a single compound. This type of library may be used to screen for improved versions of the compound, for example, for a compound variant having optimized therapeutic efficacy.

For these or any other application, general approaches for increasing library diversity are very useful and represent an important focus of the protein design industry.

### Summary of the Invention

In general, the present invention features a method for generating a nucleic acid library, the method involving: (a) providing a population of single-stranded nucleic acid templates, each of the templates including a coding sequence and an operably linked promoter sequence; (b) hybridizing to the population of single-stranded nucleic acid templates a mixture of substantially complementary single-stranded nucleic acid fragments, the fragments being shorter in length than the nucleic acid template; (c) contacting each of the hybridization products of step (b) with both a DNA polymerase which lacks strand displacement activity and a DNA ligase under conditions in which the fragments act as primers for the completion of a second nucleic acid strand which is substantially complementary to the nucleic acid template; and (d) contacting the products of step (c) with RNA polymerase to generate an RNA library, the library being transcribed from the second nucleic acid strand.

In preferred embodiments, the method is used to introduce one or more mutations into the library; the mixture of substantially complementary single-stranded nucleic acid fragments is generated by cleaving a double-stranded nucleic acid molecule; the mixture of substantially complementary single-stranded nucleic acid fragments is generated by synthesis of random oligonucleotides; the single-stranded nucleic acid template is generated using an M13 phage carrying the nucleic acid, by digestion of one strand of a double-stranded nucleic acid template using gene VI exonuclease or lambda exonuclease, by capture of a biotinylated single nucleic acid strand using streptavidin, or by reverse transcription of RNA; the mixture of substantially complementary single-stranded nucleic acid fragments includes at least about 100 different species of nucleic acid fragments; step (b) is carried out using between 1 and approximately 1000 fragments per single-stranded nucleic acid template; a single strand of the product of step (c) is used as a nucleic acid template and steps (b) and (c) are repeated; steps (b) and (c) are repeated, using, in each round, the product of step (c) as the nucleic acid template; the method further involves providing one or more single-stranded nucleic acid fragments which form a homoduplex with the single-stranded nucleic acid template and carrying out step (b) in the presence of the homoduplex-forming fragments; the promoter is a T7 promoter; the DNA polymerase is T4 DNA polymerase; the method further involves amplifying the product of step (c) prior to said contacting step (d); the method further involves the step of: (e) translating the RNA library to generate a protein library; the method further involves the step of: (e) linking to the 3' terminus of the coding sequence of each of substantially all of the members of the RNA library an amino acid acceptor molecule; and the method further involves the step of (f) translating the RNA library to generate an RNA-protein fusion library.

In a second aspect, the invention features a method for reducing sequence variation in a population of nucleic acid molecules, the method involving: (a) providing a first population of single-stranded nucleic acid templates of varying sequence, each of substantially all of the templates including a coding sequence and an operably linked promoter sequence; (b) hybridizing to the members of the first population a second population of substantially complementary single-stranded nucleic acid fragments, the fragments being shorter in length than the nucleic acid template and the fragments being of substantially identical sequence; (c) contacting the hybridization products of step (b) with both a DNA polymerase which lacks strand displacement activity and a DNA ligase under conditions in which the fragments act as primers for the completion of a second nucleic acid strand which is substantially complementary to the nucleic acid template; and (d) contacting the products of step (c) with RNA polymerase to generate a population of RNA molecules, the population of RNA molecules being transcribed from the second nucleic acid strand and having reduced sequence variation relative to the first population of single-stranded nucleic acid templates.

In preferred embodiments, the method is used to remove one or more mutations from the first population of single-stranded nucleic acid templates; step (b) involves hybridization of the first population of single-stranded nucleic acid templates to two or more different populations of substantially complementary single-stranded nucleic acid fragments; the second population of substantially complementary single-stranded nucleic acid fragments is generated by cleaving a double-stranded nucleic acid molecule; the second population of substantially complementary single-stranded nucleic acid fragments is generated by synthesis of random oligonucleotides; the single-stranded nucleic acid template is generated using an M13 phage carrying the nucleic acid, by digestion of one strand of a double-stranded nucleic acid template using gene VI exonuclease or lambda exonuclease, by capture of a biotinylated single nucleic acid strand using streptavidin, or by reverse transcription of RNA, step (b) is carried out using between 1 and approximately 1000 single-stranded nucleic acid fragments per single-stranded nucleic acid template; a single strand of the product of step (c) is used as a nucleic acid template and steps (b) and (c) are repeated; steps (b) and (c) are repeated, using, in each round, the product of step (c) as the nucleic acid template; the promoter is a T7 promoter; the DNA polymerase is T4 DNA polymerase; the method further involves amplifying the product of step (c) prior to said contacting step (d); the method further involves the step of: (e) translating the population of RNA molecules to generate a protein library; the method further involves the step of: (e) linking to the 3' terminus of the coding sequence of each of substantially all of the members of the population of RNA molecules an amino acid acceptor molecule; and the method further involves the step of: (f) translating the population of RNA molecules to generate an RNA-protein fusion library.

In a third aspect, the invention features a method for generating a nucleic acid library, the method involving: (a) providing a population of single-stranded nucleic acid templates, each of the templates including a coding sequence; (b) providing a population of single-stranded nucleic acid molecules of varying sequence, the population of single-stranded nucleic acid templates and the population of single-stranded nucleic acid molecules of varying sequence being substantially complementary; (c) hybridizing the population of single-stranded nucleic acid templates with the population of single-stranded nucleic acid molecules of varying sequence under conditions sufficient to form duplexes; and (d) contacting the duplexes with one or more excision/repair enzymes under conditions that allow the enzymes to correct mismatched base pairs in the duplexes.

In preferred embodiments, the method further involves providing a population of single-stranded templates derived from the product of step (d) and repeating steps (c) and (d); and the steps (c) and (d) are repeated, using, in each round, a population of single-stranded templates derived from the product of step (d).

In a fourth aspect, the invention features a method for generating a nucleic acid library, the method involving: (a) providing a population of single-stranded nucleic acid templates, each of the templates including a coding sequence; (b) hybridizing to the population of single-stranded nucleic acid templates a mixture of substantially complementary single-stranded nucleic acid fragments, the fragments being shorter in length than the nucleic acid template; (c) contacting each of the hybridization products of step (b) with both a DNA polymerase which lacks strand displacement activity and a DNA ligase under conditions in which the fragments act as primers for the completion of a second nucleic acid strand which is substantially complementary to the nucleic acid template; and (d) contacting the products of step (c) with one or more excision/repair enzymes under conditions that allow the enzymes to correct mismatched base pairs in the products.

In preferred embodiments, the method further involves providing a population of single-stranded templates derived from the product of step (d) and repeating steps (b) - (d); and steps (b) - (d) are repeated, using, in each round, a population of single-stranded templates derived from the product of step (d).

In preferred embodiments of the third and fourth aspects of the invention, the contacting with the excision/repair enzymes is carried out in vivo (for example, in a bacterial cell); the contacting with the excision/repair enzymes is carried out in vitro; the single-stranded nucleic acid template is generated using an M13 phage carrying the nucleic acid, by digestion of one strand of a double-stranded nucleic acid template using gene VI exonuclease or lambda exonuclease, by capture of a biotinylated single nucleic acid strand using streptavidin, or by reverse transcription of RNA; step (b) is carried out using between 1 and approximately 1000 single-stranded nucleic acid molecules of varying sequence or single-stranded nucleic acid fragments per single-stranded nucleic acid template; the method further involves the step of: (e) amplifying the product of step (d); each of the coding sequences is operably linked to a promoter sequence; the method further involves the step of: (e) transcribing the products of step (d) to generate an RNA library; the method further involves the step of: (f) translating the RNA library to generate a protein library; the method further involves the step of: (f) linking to the 3' terminus of the coding sequence of each of substantially all of the members of the RNA library an amino acid acceptor molecule; and the method further involves the step of: (g) translating the RNA library to generate an RNA-protein fusion library.

As used herein, by a "library" is meant at least 10⁸, preferably, at least 10¹⁰, more preferably, at least 10¹², and, most preferably, at least 10¹⁴ molecules having a nucleic acid and/or an amino acid component.

By a "mixture" of nucleic acid fragments is meant at least 100, preferably, at least 500, more preferably, at least 1000, and, most preferably, at least 1500 nucleic acid fragments.

By a "promoter sequence" is meant any nucleic acid sequence which provides a functional RNA polymerase binding site and which is sufficient to allow transcription of a proximal coding sequence.

By "substantially complementary" is meant that a nucleic acid strand possesses a sufficient number of nucleotides which are capable of forming matched Watson-Crick base pairs with a second nucleic acid strand to produce one or more regions of double-strandedness between the two nucleic acids. It will be understood that each nucleotide in a nucleic acid molecule need not form a matched Watson-Crick base pair with a nucleotide in an opposing strand to be substantially complementary, and that in a "mixture of substantially complementary single-stranded nucleic acid fragments," a significant fraction of the fragments will contain one or more nucleotides which form mismatches with the "single-stranded nucleic acid template."

By "strand displacement activity" is meant the ability of a polymerase or its associated helicase to disrupt base pairing between two nucleic acid strands.

By "mutation" is meant any nucleotide change and includes sequence alterations that result in phenotypic differences as well as changes which are silent.

By "duplex" is meant is meant a structure formed between two annealed nucleic acid strands in which sufficient sequence complementarity exists between the strands to maintain a stable hybridization complex. A duplex may be either a "homoduplex," in which all of the nucleotides in the first strand appropriately base pair with all of the nucleotides in the second opposing strand, or a heteroduplex. By a "heteroduplex" is meant a structure formed between two annealed strands of nucleic acid in which one or more nucleotides in the first strand do not or cannot appropriately base pair with one or more nucleotides in the second opposing complementary strand because of one or more mismatches. Examples of different types of heteroduplexes include those which exhibit an exchange of one or several nucleotides, and insertion or deletion mutations.

By "random oligonucleotides" is meant a mixture of oligonucleotides having sequence variation at one or more nucleotide positions. Random oligonucleotides may be produced using entirely random or partially random synthetic approaches or by intentionally altering an oligonucleotide in a directed fashion.

By an "amino acid acceptor molecule" is meant any molecule capable of being added to the C-terminus of a growing protein chain by the catalytic activity of the ribosomal peptidyl transferase function. Typically, such molecules contain (i) a nucleotide or nucleotide-like moiety (for example, adenosine or an adenosine analog (di-methylation at the N-6 amino position is acceptable)), (ii) an amino acid or amino acid-like moiety (for example, any of the 20 D- or L-amino acids or any amino acid analog thereof (for example, O-methyl tyrosine or any of the analogs described by Ellman et al., Meth. Enzymol. 202:301, 1991)), and (iii) a linkage between the two (for example, an ester, amide, or ketone linkage at the 3' position or, less preferably, the 2' position); preferably, this linkage does not significantly perturb the pucker of the ring from the natural ribonucleotide conformation. Amino acid acceptors may also possess a nucleophile, which may be, without limitation, an amino group, a hydroxyl group, or a sulfhydryl group. In addition, amino acid acceptors may be composed of nucleotide mimetics, amino acid mimetics, or mimetics of a combined nucleotide-amino acid structure.

By an amino acid acceptor being linked "to the 3' terminus" of a coding sequence is meant that the amino acid acceptor molecule is positioned after the final codon of that coding sequence. This term includes, without limitation, an amino acid acceptor molecule that is positioned precisely at the 3' end of the coding sequence as well as one which is separated from the final codon by intervening coding or non-coding sequence (for example, a sequence corresponding to a pause site). This term also includes constructs in which coding or non-coding sequences follow (that is, are 3' to) the amino acid acceptor molecule. In addition, this term encompasses, without limitation, an amino acid acceptor molecule that is covalently bonded (either directly or indirectly through intervening nucleic acid sequence) to the coding sequence, as well as one that is joined to the coding sequence by some non-covalent means, for example, through hybridization using a second nucleic acid sequence that binds at or near the 3' end of the coding sequence and that itself is bound to an amino acid acceptor molecule.

By an "RNA-protein" fusion is meant any molecule that includes a ribonucleic acid covalently bonded through an amide bond to a protein. This covalent bond is resistant to cleavage by a ribosome.

By a "protein" is meant any two or more naturally occurring or modified amino acids joined by one or more peptide bonds. "Protein," "peptide," and "polypeptide" are used interchangeably herein.

By "a population of single-stranded templates of varying sequence" is meant that the nucleic acid species of the population possess sequences which differ at one or more nucleotide positions.

By "excision/repair enzymes" is meant any combination of enzymes sufficient to replace a mismatched base pair or loop with a standard base pair (i.e., A:T or G:C).

### Brief Description of the Drawings

FIGURE 1 is a schematic representation of an exemplary fragment recombination method for generating highly diverse RNA-protein fusion libraries. In this method, the fragments are derived from a double-stranded DNA molecule into which sequence variation is introduced.
FIGURE 2 is a schematic representation of the initial steps of a second exemplary fragment recombination method. In this method, the fragments are synthetic oligonucleotides into which sequence variation is introduced.

### Detailed Description

The present invention involves a number of novel and related methods for the random recombination of nucleic acid sequences, facilitating the generation of DNA, RNA, and protein libraries into which genetic alterations have been introduced. As described in more detail below, in one preferred embodiment, this technique is carried out in vitro and is used to generate traditional protein libraries or RNA-protein fusion libraries, either of which may then be used in combination with any of a variety of methods for the selection of desired proteins or peptides (or their corresponding coding sequences) from library populations. This general approach provides a means for the introduction of mutations into protein libraries in an unbiased fashion and also provides a technique by which unfavorable mutations may be removed from a library or selected pool, or "backcrossed" out of a population of molecules during subsequent rounds of selection.

### Fragment Recombination

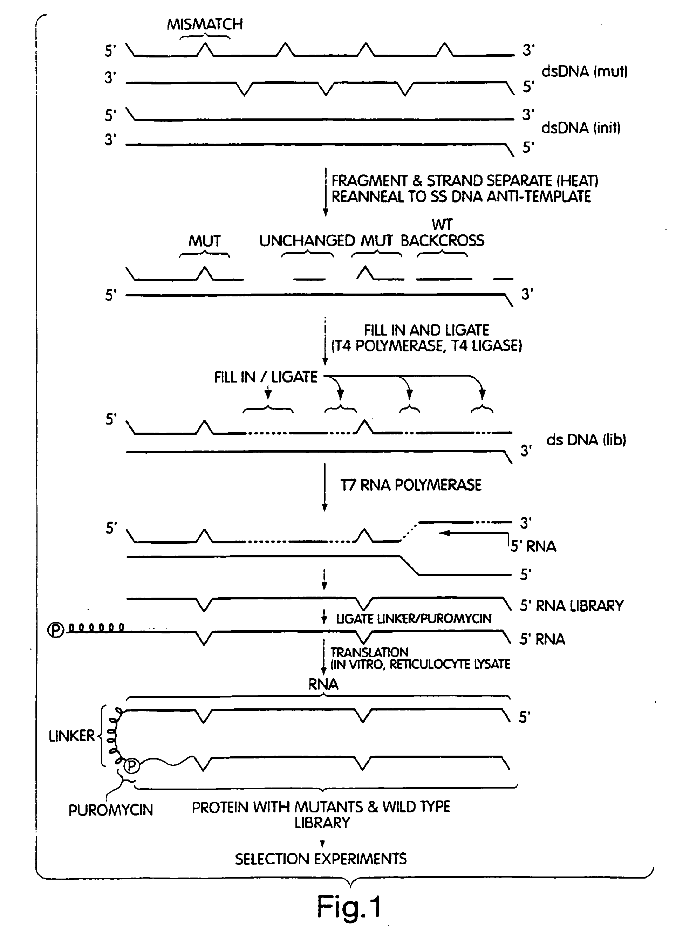

According to one preferred method of the invention, a library is generated by the production of mutant fragments and the random recombination of these fragments with an unmutated (typically, wild-type) sequence. One example of this general approach is shown in Figure 1. As indicated in this figure, mutations are first randomly introduced into an initial double-stranded DNA sequence (termed "dsDNA(init)"). This produces a population of mutant double-stranded DNA sequences, which, in Figure 1, is termed "dsDNA(mut)." These mutations may be introduced by any technique, including PCR mutagenesis (which relies on the poor error-proofing mechanism of Taq polymerase), site-directed mutagenesis, or template-directed mutagenesis (for example, as described in Joyce and Inoue, Nucl. Acids Res. 17:171, 1989. The DNA in this mutation-containing population is subsequently fragmented using any of a variety of standard methods. For example, the DNA may be partially degraded using one or more nucleases (such as DNase I, micrococcal nuclease, restriction endonucleases, or P1 nuclease), or may be fragmented chemically using, for example, Fe•EDTA. Alternatively, mutation-containing fragments may be generated by limited nucleotide consumption during polymerization (for example, during PCR amplification), or by simple physical shearing (for example, by sonication). Preferable fragment sizes range from 25-1000 base pairs and are most preferably in the range of 50-150 base pairs.

WO97/20078 describes the random fragmentation of double-stranded DNA and subsequent reassembly of said fragments in a PCR-like reaction to form recombinant double-stranded DNA.

In the present invention, the DNA fragments are heated and subsequently annealed to a full-length single-stranded DNA template which is identical to the initial DNA. in sequence and which is the non-coding (or minus) strand of that DNA. In addition, in this hybridization mixture is included a second type of fragment, sometimes referred to as a "terminator fragment" (Joyce and Inoue, Nucl. Acids Res. 17:171, 1989). This terminator fragment is complementary to the 3' end of the single-stranded template and provides a polymerization primer which binds to the template in a manner that is relatively independent of the number or nature of the randomly annealed, mutation-containing fragments.

Single-stranded templates may be generated by any standard technique, for example, by using an M 13 phage carrying the DNA sequence, by digestion of the coding strand of a dsDNA(init) molecule using gene VI exonuclease (Nikiforov et al, PCR Methods Appl. 3:285, 1994) or lambda exonuclease (Higuchi and Ochman, Nucl. Acids Res 17: 5865, 1989), by capture of a biotinylated DNA strand using immobilized streptavidin (Joyce and Inoue, Nucl. Acids Res. 17:171, 1989), or by reverse transcription ofRNA. To carry out the template-fragment hybridization, templates are mixed with fragments using no less than one fragment molecule per template molecule and no more than approximately 1000 fragment molecules per template molecule. A low ratio of fragments to templates produces progeny strands that closely resemble the templates, whereas a higher ratio produces progeny that more closely resemble the fragments. Hybridization conditions are determined by standard methods and are designed to allow for the formation of heteroduplexes between the template and the fragments. Exemplary hybridization techniques are described, for example, in Stemmer. U.S. Patent No. 5,605,793.

Once annealed to the template, the fragments are joined together by treating with both a DNA polymerase that lacks strand displacement activity and a DNA ligase. DNA polymerases useful for this purpose include, without limitation, T4 DNA polymerase and reconstituted DNA pol II from E. coli (see, for example, Hughes et al., J. Biol. Chem. 266:4568, 1991). Any DNA ligase (for example, T4 DNA ligase) may be utilized. In this step, the DNA duplexes may be treated first with the DNA polymerase and then with the DNA ligase, or with both enzymes simultaneously, and the step may be carried out, for example, as described in Joyce and Inoue (Nucl. Acids Res. 17:711, 1989). As shown in Figure 1, this step generates a population of double-stranded DNAs (termed "dsDNA(lib)), each member of which includes one strand typically having one or more introduced mutations. Because both the mutations initially introduced and the number and nature of the fragments annealed are random, different duplexes in the population contain different mutant sequences.

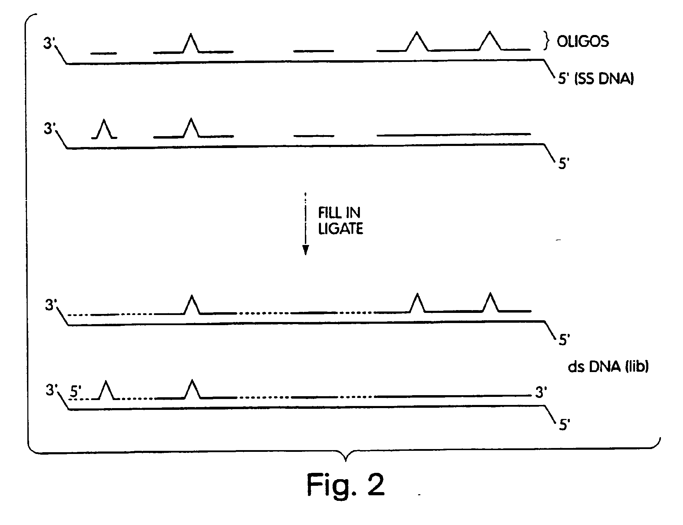

An alternative to this general approach for generating a double-stranded DNA library is shown in Figure 2. By this alternative approach, single-stranded oligonucleotide fragments are synthesized which correspond to portions of the coding strand of an initial double-stranded DNA molecule. These oligonucleotide fragments preferably range from 5-2000 nucleotides, and most preferably range from 20-100 nucleotides in length and are generated, for example, using any standard technique of nucleic acid synthesis. These oligonucleotides may be synthesized with completely random or semi-random mutations by any standard technique. Preferably, such oligonucleotides include up to 3 introduced mismatches per 20 nucleotide segment and are devoid of in frame stop codons. In addition, in certain cases, it may be desirable or necessary to increase the hybridization potential of the oligonucleotide through the introduction of non-natural, affinity-enhancing base pairs, such as C-5 propyne uridine or C-5 propyne cytidine. These techniques are described, for example, in Wagner et al., Science 260:1510, 1993.

These mutation-containing oligonucleotide fragments are next annealed to single-stranded templates which, as above, are full-length strands identical in sequence to the non-coding (or minus) strand of the initial DNA. The fragments are joined together using DNA polymerase and DNA ligase, also as described above, to create a double-stranded DNA library (dsDNA(lib)). Again, this library contains a population of duplex molecules, containing an array of different coding strands having mutations which differ in number, position, and identity.

If desired, the above steps may be repeated, for either the fragment or the oligonucleotide approach, to introduce varying numbers of mutations into a DNA molecule. In particular, the mutated strands become the initial single-stranded templates, and mutant fragments or oligonucleotides are annealed to those strands and polymerized and ligated.

### Methods for Backcrossing

As generally described above, the methods of the invention are used to introduce mutations into an initial DNA sequence. In addition, these techniques may be used to remove or reduce in frequency undesirable mutations from a DNA library. According to this approach, following fragmentation of the dsDNA(mut), oligonucleotides of wild-type sequence or specific fragments of unmutated or wild-type DNA (wtDNA) may be added to the single-stranded template together with the dsDNA(mut) fragments or oligonucleotides. The fragments are strand-separated (if necessary), annealed to the full-length single-stranded template, and joined together using DNA polymerase and DNA ligase, as described above. The use of a high concentration of unmutated oligonucleotide or fragment, relative to the corresponding mutant fragment, allows for the generation of libraries in which undesirable mutations are minimized or eliminated.

In addition, this approach may be used with existing mutation-containing libraries to similarly decrease or eliminate undesirable sequences. This approach involves an initial library having mutant sequences and the annealing, polymerization, and ligation of fragments or oligonucleotides of wild-type sequence, as generally described above.

### RNA, Protein, and RNA-Protein Libraries

In one preferred embodiment of the invention, the DNA libraries described above further include an RNA polymerase binding site, for example, for T7 or SP6 polymerase. Such binding sites are described, for example, in Milligan et al., Proc. Natl. Acad. Sci. USA 87:696, 1990. This site is positioned upstream of the coding sequence at a location which allows for transcription of the sequence. Typically such sites are located at between 5-2000 base pairs upstream of the coding sequence.

Libraries containing RNA polymerase binding sites may be altered as described above. Following polymerization and ligation, the dsDNA(lib) may be transcribed directly, for example, using an in vitro transcription system, to generate an RNA library. Alternatively, the dsDNA(lib) may be transcribed and translated directly, for example, using in vitro transcription and translation systems, to generate a protein library. Exemplary in vitro transcription systems and in vitro translation systems include T7 transcription systems, and rabbit reticulocyte, wheat germ, yeast, and E. coli translation systems.

If desired, the number of copies of each RNA or protein in the library may be increased by including a strand-specific amplification step prior to transcription. For example, PCR amplification may be carried out by incorporating unique primer-binding sequences are incorporated into the mutant strand during the polymerization and ligation steps. These sequences may be incorporated as either mismatches or sequence extensions at one or both ends of the DNA, allowing amplification of the newly-synthesized strand without amplification of the template strand. Alternatively, linear amplification can be achieved by multiple cycles of annealing and extension of a single oligonucleotide primer that is complementary to the 3' end of the newly-synthesized strand. Subsequent PCR and transcription steps produce a majority of RNA corresponding to mutant sequences with only a small proportion of template-derived sequences.

In one preferred approach, the above methods for introducing mutations or for backcrossing out undesirable mutations may be used to produce highly diverse RNA-protein libraries. Such libraries may be constructed by ligating linkers containing a non-hydrolyzable amino acid acceptor molecule, such as puromycin, to the 3' termini of the RNAs in a library (for example, produced as described above). Exemplary techniques for generating RNA-protein fusions are described, for example, in Szostak et al., U.S.S.N. 09/007,005; and Roberts et al., Proc. Natl. Acad. Sci. USA 94:12297, 1997. Subsequent translation of these RNAs generates a library of RNA-protein fusion molecules that may subsequently be used in in vitro selection experiments.

In addition, if desired, RNA or RNA-protein fusion molecules, once selected, may be used as templates in standard PCR reactions to obtain the corresponding coding sequence. Thus, this method provides a means for carrying out fragment recombination, molecular backcrossing, selection of proteins and/or peptides, and selection of their corresponding coding sequences, all in an in vitro system.

### Excision/Repair

In addition to fragment recombination approaches, excision/repair may also be used to alter library sequences. This approach may be used to generate DNA, RNA, and RNA-protein fusion libraries. This technique relies on the fact that the dsDNA(lib)s, produced by any of the methods described above, by their nature, contain a certain number of mismatched base pairs. To generate diversity in the library sequences, these mismatches are repaired in vitro by excision/repair enzymes. This may be carried out using any excision repair system (for example, as described in Jaiswal et al., Nucl. Acids Res. 26:2184, 1998; or Fortini et al., Biochemistry 37:3575, 1998.

Alternatively, the excision/repair step may be carried out by transforming a dsDNA(lib) into a bacterial or yeast strain and exploiting the bacterial or yeast repair systems in vivo. Again, this step may be carried out by transforming the library into any standard in vivo excision/repair system. Exemplary systems are described, without limitation, in Campbell et al., Mutat. Res. 211:181, 1989; Bishop and Kolodner, Mol. Cell Biol. 6:3401, 1986; Fishel et al., J. Mol. Biol. 188:147, 1986; and Westmoreland et al., Genetics 145:29, 1997.

Because the above repair processes are random, this excision/repair method sometimes results in the introduction of mutations into a library sequence and at other times results in the backcrossing of wild-type sequence alterations into the coding strand.

In an alternative to the above approaches, in vitro or in vivo excision/repair may also be used directly to generate diverse libraries using as a substrate a mixture of dsDNA(mut) (for example, produced as described above) and dsDNA(init) or wtDNA. In this technique, the mixture is strand-separated and reannealed, and is then either incubated in vitro with excision/repair enzymes or transformed into bacteria to utilize the bacterial excision/repair system (for example, as described above). In this manner, mutations may be randomly introduced into a sequence, and wild-type sequences may be backcrossed into dsDNA(mut) molecules.

## Claims

1. A method for generating a nucleic acid library, said method comprising:
(a) providing a population of single-stranded nucleic acid templates, each of said templates comprising a coding sequence and an operably linked promoter sequence;
(b) hybridizing to said population of single-stranded nucleic acid templates a mixture of substantially complementary single-stranded nucleic acid fragments, said fragments being shorter in length than said nucleic acid templates;
(c) contacting each of the hybridization products of step (b) with both a DNA polymerase which lacks strand displacement activity and a DNA ligase under conditions in which said fragments randomly annealed act as primers for the completion of second nucleic acid strands which are substantially complementary to said nucleic acid templates, thereby producing a population of coding strands having mutations, which coding strands differ from one another in number, position, and identity of said mutations; and
(d) contacting the products of step (c) with RNA polymerase to generate an RNA library, said library being transcribed from said second nucleic acid strands.

2. The method of claim 1, wherein said method is used to introduce one or more mutations into said library.

3. A method for reducing sequence variation in a population of nucleic acid molecules, said method comprising:
(a) providing a first population of single-stranded nucleic acid templates of varying sequence, each of substantially all of said templates comprising a coding sequence and an operably linked promoter sequence;
(b) hybridizing to the members of said first population a second population of single-stranded nucleic acid fragments, said fragments being shorter in length than said nucleic acid templates and said fragments being substantially complementary to said nucleic acid templates, wherein a subpopulation of said second population comprises a substantially identical sequence and hybridizes to a region of varying sequence;
(c) contacting the hybridization products of step (b) with both a DNA polymerase which lacks strand displacement activity and a DNA ligase under conditions in which said fragments act as primers for the completion of second nucleic acid strands which are substantially complementary to said nucleic acid templates; and
(d) contacting the products of step (c) with RNA polymerase to generate a third population of RNA molecules, said third population of RNA molecules being transcribed from said second nucleic acid strands and having reduced sequence variation relative to said first population of single-stranded nucleic acid templates.

4. The method of claim 3, wherein said method is used to remove one or more mutations from said first population of single-stranded nucleic acid templates.

5. The method of claim 1 or 3, wherein said mixture of substantially complementary single-stranded nucleic acid fragments is generated by cleaving double- stranded nucleic acid molecules or by synthesis of random oligonucleotides.

6. The method of claim 1 or 3, wherein said mixture of substantially complementary single-stranded nucleic acid fragments comprises at least about 100 different species of nucleic acid fragments.

7. The method of claim 1 or 3, further comprising generating complementary strands from said second nucleic acid strands, providing said complementary strands as said population or said first population of single-stranded nucleic acid templates, and repeating steps (b) and (c) for one or more rounds, and then repeating step (d).

8. The method of claim 1, wherein said method further comprises providing one or more single-stranded nucleic acid fragments which form a homoduplex with said single-stranded nucleic acid templates and carrying out step (b) in the presence of said homoduplex-forming fragments.

9. The method of claim 1 or 3, wherein said promoter is a T7 promoter.

10. The method of claim 1 or 3, wherein said DNA polymerase is T4 DNA polymerase.

11. The method of claim 1 or 3, wherein said method further comprises amplifying said product of step (c) prior to said contacting step (d).

12. The method of claim 1 or 3, further comprising a method to generate a protein library, wherein said method comprises the step of:
(e) translating said RNA library to generate a protein library.

13. The method of claim 1 or 3, further comprising a method to generate an RNA-protein fusion library, wherein said method comprises the steps of:
(e) linking an amino acid acceptor molecule to the 3' terminus of said coding sequence of each of substantially all of the members of said RNA library; and optionally
(f) translating said RNA library to generate an RNA-protein fusion library.

14. A method for generating a nucleic acid library, said method comprising:
(a) providing a first population of single-stranded nucleic acid templates, each of said templates comprising a coding sequence;
(b) providing a second population of single-stranded nucleic acid molecules of varying sequence, said first population of single-stranded nucleic acid templates and said second population of single-stranded nucleic acid molecules of varying sequence being substantially complementary;
(c) hybridizing said first population of single-stranded nucleic acid templates with said second population of single-stranded nucleic acid molecules of varying sequence under conditions sufficient to form duplexes; and
(d) contacting said duplexes with one or more excision/repair enzymes under conditions that allow said enzymes to correct mismatched base pairs in said duplexes.

15. The method of claim 14, wherein said method further comprises, after step (d), providing a new population of single-stranded nucleic acid templates comprising a coding sequence, said templates being derived from the product of step (d), substituting said new population of templates for said first population of templates, and repeating steps (c), and (d) for one or more rounds.

16. A method for generating a nucleic acid library, said method comprising:
(a) providing a population of single-stranded nucleic acid templates, each of said templates comprising a coding sequence;
(b) hybridizing to said population of single-stranded nucleic acid templates a mixture of substantially complementary single-stranded nucleic acid fragments, said fragments being shorter in length than said nucleic acid templates;
(c) contacting each of the hybridization products of step (b) with both a DNA polymerase which lacks strand displacement activity and a DNA ligase under conditions in which said fragments act as primers for the completion of a second nucleic acid strand which is substantially complementary to said nucleic acid templates; and
(d) contacting the products of step (c) with one or more excision/repair enzymes under conditions that allow said enzymes to correct mismatched base pairs in said products.

17. The method of claim 16, wherein said method further comprises the steps of: providing a new population of single-stranded nucleic acid templates comprising a coding sequence and derived from the product of step (d), substituting said new population of templates for said population of single-stranded nucleic acid templates and repeating steps (b)-(d) for one or more rounds.

18. The method of claim 14 or 16 wherein said contacting with said excision/repair enzymes is carried out *in vitro.*

19. The method of claim 14 or 16, wherein said contacting with said excision/repair enzymes is carried out in a bacterial cell.

20. The method of claim 1, 3, 14 or 16 wherein said single-stranded nucleic acid templates are generated by using an M13 phage carrying said nucleic acid templates, by digestion of one strand of double-stranded nucleic acid templates using gene VI exonuclease or lambda exonuclease, by capture of biotinylated single nucleic acid strands using streptavidin, or by reverse transcription of RNA.

21. The method of claim 1, 3, 14 or 16, wherein step (b) is carried out using between 1 and approximately 1000 single-stranded nucleic acid molecules of varying sequence or single-stranded nucleic acid fragments per each of said single-stranded nucleic acid templates.

22. The method of claim 14 or 16, wherein said method further comprises the step of:
(e) amplifying said product of step (d).

23. The method of claim 14 or 16, wherein each of said coding sequences is operably linked to a promoter sequence.

24. The method of claim 23, wherein said method further comprises the step of:
(e) transcribing the products of step (d) to generate an RNA library.

25. The method of claim 24, further comprising a method to generate a protein library, wherein said method comprises the step of:
(f) translating said RNA library to generate a protein library.

26. The method of claim 24, further comprising a method to generate a an RNA-portion fusion library, wherein said method comprises the steps of:
(f) linking an amino acid acceptor molecule to the 3' terminus of said coding sequence of each of substantially all of the members of said RNA library; and optionally
(g) translating said RNA library to generate an RNA-protein fusion library.

## Patentansprüche

1. Verfahren zum Generieren einer Nucleinsäurebibliothek, wobei das Verfahren umfasst:
(a) Bereitstellen einer Population von einzelsträngigen Nucleinsäuretemplaten, wobei jedes der Template eine Codierungssequenz und eine operabel verknüpfte Promotorsequenz umfasst;
(b) Hybridisieren eines Gemisches von im Wesentlichen komplementären einzelsträngigen Nucleinsäurefragmenten mit der Population von einzelsträngigen Nucleinsäuretemplaten, wobei die Fragmente in der Länge kürzer als die Nucleinsäuretemplate sind;
(c) Inkontaktbringen von jedem der Hybridisierungsprodukte von Schritt (b) mit sowohl einer DNA-Polymerase, der Strangverdrängungsaktivität fehlt, als auch einer DNA-Ligase unter Bedingungen, bei welchen die Fragmente statistisch anneliert als Primer für die Vollendung von zweiten Nucleinsäuresträngen wirken, welche im Wesentlichen komplementär zu den Nucleinsäuretemplaten sind, dadurch Erzeugen einer Population von Codierungssträngen mit Mutationen, welche Codierungsstränge sich in der Anzahl, Position und Identität der Mutationen voneinander unterscheiden; und
(d) Inkontaktbringen der Produkte von Schritt (c) mit RNA-Polymerase, um eine RNA-Bibliothek zu generieren, wobei die Bibliothek von den zweiten Nucleinsäuresträngen transkribiert wird.

2. Verfahren nach Anspruch 1, wobei das Verfahren verwendet wird, um eine oder mehrere Mutationen in die Bibliothek einzuführen.

3. Verfahren zum Verringern von Sequenzvariation in einer Population von Nucleinsäuremolekülen, wobei das Verfahren umfasst:
(a) Bereitstellen einer ersten Population von einzelsträngigen Nucleinsäuretemplaten variierender Sequenz, wobei jedes von im Wesentlichen allen der Template eine Codierungssequenz und eine operabel verknüpfte Promotorsequenz umfasst;
(b) Hybridisieren einer zweiten Population von einzelsträngigen Nucleinsäurefragmenten mit den Mitgliedern der ersten Population, wobei die Fragmente in der Länge kürzer als die Nucleinsäuretemplate sind und wobei die Fragmente im Wesentlichen komplementär zu den Nucleinsäuretemplaten sind, wobei eine Subpopulation der zweiten Population eine im Wesentlichen identische Sequenz umfasst und mit einer Region variierender Sequenz hybridisiert;
(c) Inkontaktbringen der Hybridisierungsprodukte von Schritt (b) mit sowohl einer DNA-Polymerase, der Strangverdrängungsaktivität fehlt, als auch einer DNA-Ligase unter Bedingungen, bei welchen die Fragmente als Primer für die Vollendung von zweiten Nucleinsäuresträngen wirken, welche im Wesentlichen komplementär zu den Nucleinsäuretemplaten sind; und
(d) Inkontaktbringen der Produkte von Schritt (c) mit RNA-Polymerase, um eine dritte Population von RNA-Molekülen zu generieren, wobei die dritte Population von RNA-Molekülen von den zweiten Nucleinsäuresträngen transkribiert wird und verringerte Sequenzvariation relativ zu der ersten Population von einzelsträngigen Nucleinsäuretemplaten aufweist.

4. Verfahren nach Anspruch 3, wobei das Verfahren verwendet wird, um eine oder mehrere Mutationen aus der ersten Population von einzelsträngigen Nucleinsäuretemplaten zu entfernen.

5. Verfahren nach Anspruch 1 oder 3, wobei das Gemisch von im Wesentlichen komplementären einzelsträngigen Nucleinsäurefragmenten durch Spalten von doppelsträngigen Nucleinsäuremolekülen oder durch Synthese von statistischen Oligonucleotiden generiert wird.

6. Verfahren nach Anspruch 1 oder 3, wobei das Gemisch von im Wesentlichen komplementären einzelsträngigen Nucleinsäurefragmenten mindestens etwa 100 unterschiedliche Spezies von Nucleinsäurefragmenten umfasst.

7. Verfahren nach Anspruch 1 oder 3, weiterhin umfassend Generieren von komplementären Strängen aus den zweiten Nucleinsäuresträngen, Bereitstellen der komplementären Stränge als der Population oder der ersten Population von einzelsträngigen Nucleinsäuretemplaten und Wiederholen der Schritte (b) und (c) für eine oder mehrere Runden und dann Wiederholen von Schritt (d).

8. Verfahren nach Anspruch 1, wobei das Verfahren weiterhin Bereitstellen von einem oder mehreren einzelsträngigen Nucleinsäurefragmenten, welche einen Homoduplex mit den einzelsträngigen Nucleinsäuretemplaten bilden, und Ausführen von Schritt (b) in Gegenwart der Homoduplex-bildenden Fragmente umfasst.

9. Verfahren nach Anspruch 1 oder 3, wobei der Promotor ein T7-Promotor ist.

10. Verfahren nach Anspruch 1 oder 3, wobei die DNA-Polymerase T4-DNA-Polymerase ist.

11. Verfahren nach Anspruch 1 oder 3, wobei das Verfahren weiterhin Amplifizieren des Produkts von Schritt (c) vor dem Schritt des Inkontaktbringens (d) umfasst.

12. Verfahren nach Anspruch 1 oder 3, weiterhin umfassend ein Verfahren, um eine Proteinbibliothek zu generieren, wobei das Verfahren den Schritt umfasst:
(e) Translatieren der RNA-Bibliothek, um eine Proteinbibliothek zu generieren.

13. Verfahren nach Anspruch 1 oder 3, weiterhin umfassend ein Verfahren, um eine RNA-Proteinfusionsbibliothek zu generieren, wobei das Verfahren die Schritte umfasst:
(e) Verknüpfen eines Aminosäure-Akzeptormoleküls mit dem 3'-Terminus der Codierungssequenz von jedem von im Wesentlichen allen der Mitglieder der RNA-Bibliothek; und gegebenenfalls
(f) Translatieren der RNA-Bibliothek, um eine RNA-Proteinfusionsbibliothek zu generieren.

14. Verfahren zum Generieren einer Nucleinsäurebibliothek, wobei das Verfahren umfasst:
(a) Bereitstellen einer ersten Population von einzelsträngigen Nucleinsäuretemplaten, wobei jedes der Template eine Codierungssequenz umfasst;
(b) Bereitstellen einer zweiten Population von einzelsträngigen Nucleinsäuremolekülen variierender Sequenz, wobei die erste Population von einzelsträngigen Nucleinsäuretemplaten und die zweite Population von einzelsträngigen Nucleinsäuremolekülen variierender Sequenz im Wesentlichen komplementär sind;
(c) Hybridisieren der ersten Population von einzelsträngigen Nucleinsäuretemplaten mit der zweiten Population von einzelsträngigen Nucleinsäuremolekülen variierender Sequenz unter Bedingungen, ausreichend, um Duplexe zu bilden; und
(d) Inkontaktbringen der Duplexe mit einem oder mehreren Excisions/Reparatur-Enzymen unter Bedingungen, die es den Enzymen erlauben, fehlangepasste Basenpaare in den Duplexen zu korrigieren.

15. Verfahren nach Anspruch 14, wobei das Verfahren weiterhin nach Schritt (d) Bereitstellen einer neuen Population von einzelsträngigen Nucleinsäuretemplaten, umfassend eine Codierungssequenz, wobei die Template von dem Produkt von Schritt (d) abgeleitet sind, Substituieren der neuen Population von Templaten für die erste Population von Templaten und Wiederholen der Schritte (c) und (d) für eine oder mehrere Runden umfasst.

16. Verfahren zum Generieren einer Nucleinsäurebibliothek, wobei das Verfahren umfasst:
(a) Bereitstellen einer Population von einzelsträngigen Nucleinsäuretemplaten, wobei jedes der Template eine Codierungssequenz umfasst;
(b) Hybridisieren eines Gemisches von im Wesentlichen komplementären einzelsträngigen Nucleinsäurefragmenten mit der Population von einzelsträngigen Nucleinsäuretemplaten, wobei die Fragmente in der Länge kürzer als die Nucleinsäuretemplate sind;
(c) Inkontaktbringen von jedem der Hybridisierungsprodukte von Schritt (b) mit sowohl einer DNA-Polymerase, der Strangverdrängungsaktivität fehlt, als auch einer DNA-Ligase unter Bedingungen, bei welchen die Fragmente als Primer für die Vollendung eines zweiten Nucleinsäurestranges wirken, welcher im Wesentlichen komplementär zu den Nucleinsäuretemplaten ist; und
(d) Inkontaktbringen der Produkte von Schritt (c) mit einem oder mehreren Excisions/Reparatur-Enzymen unter Bedingungen, die es den Enzymen erlauben, fehlangepasste Basenpaare in den Produkten zu korrigieren.

17. Verfahren nach Anspruch 16, wobei das Verfahren weiterhin die Schritte umfasst: Bereitstellen einer neuen Population von einzelsträngigen Nucleinsäuretemplaten, umfassend eine Codierungssequenz und abgeleitet von dem Produkt von Schritt (d), Substituieren der neuen Population von Templaten für die Population von einzelsträngigen Nucleinsäuretemplaten und Wiederholen der Schritte (b)-(d) für eine oder mehrere Runden.

18. Verfahren nach Anspruch 14 oder 16, wobei das Inkontaktbringen mit den Excisions/Reparatur-Enzymen *in vitro* ausgeführt wird.

19. Verfahren nach Anspruch 14 oder 16, wobei das Inkontaktbringen mit den Excisions/Reparatur-Enzymen in einer Bakterienzelle ausgeführt wird.

20. Verfahren nach Anspruch 1, 3, 14 oder 16, wobei die einzelsträngigen Nucleinsäuretemplate durch Verwendung eines M13-Phagen, tragend die Nucleinsäuretemplate, durch Digestion eines Stranges von doppelsträngigen Nucleinsäuretemplaten unter Verwendung von Gen-VI-Exonuclease oder lambda-Exonuclease, durch Einfang von biotinylierten einzelnen Nucleinsäuresträngen unter Verwendung von Streptavidin oder durch reverse Transkription von RNA generiert werden.

21. Verfahren nach Anspruch 1, 3, 14 oder 16, wobei Schritt (b) ausgeführt wird, indem zwischen 1 und ungefähr 1000 einzelsträngige Nucleinsäuremoleküle variierender Sequenz oder einzelsträngige Nucleinsäurefragmente für jedes der einzelsträngigen Nucleinsäuretemplate verwendet werden.

22. Verfahren nach Anspruch 14 oder 16, wobei das Verfahren weiterhin den Schritt umfasst:
(e) Amplifizieren des Produkts von Schritt (d).

23. Verfahren nach Anspruch 14 oder 16, wobei jede von den Codierungssequenzen operabel mit einer Promotorsequenz verknüpft ist.

24. Verfahren nach Anspruch 23, wobei das Verfahren weiterhin den Schritt umfasst:
(e) Transkribieren der Produkte von Schritt (d), um eine RNA-Bibliothek zu generieren.

25. Verfahren nach Anspruch 24, weiterhin umfassend ein Verfahren, um eine Protein-Bibliothek zu generieren, wobei das Verfahren den Schritt umfasst:
(f) Translatieren der RNA-Bibliothek, um eine Proteinbibliothek zu generieren.

26. Verfahren nach Anspruch 24, weiterhin umfassend ein Verfahren, um eine RNA-Proteinfusionsbibliothek zu generieren, wobei das Verfahren die Schritte umfasst:
(f) Verknüpfen eines Aminosäure-Akzeptormoleküls mit dem 3'-Terminus der Codierungssequenz von jedem von im Wesentlichen allen der Mitglieder der RNA-Bibliothek; und gegebenenfalls
(g) Translatieren der RNA-Bibliothek, um eine RNA-Proteinfusionsbibliothek zu generieren.

## Revendications

1. Procédé servant à générer une banque d'acides nucléiques, ledit procédé comprenant les étapes consistant à:
(a) se procurer une population de matrices d'acide nucléique monocaténaires, chacune desdites matrices comprenant une séquence codante et une séquence promotrice fonctionnellement liée;
(b) hybrider à ladite population de matrices d'acide nucléique monocaténaires un mélange de fragments d'acide nucléique monocaténaires sensiblement complémentaires, lesdits fragments étant de taille plus courte que lesdites matrices d'acide nucléique;
(c) mettre en contact chacun des produits d'hybridation de l'étape (b) à la fois avec une ADN polymérase qui est dépourvue d'activité de déplacement de brin et avec une ADN ligase dans des conditions dans lesquelles lesdits fragments aléatoirement appariés agissent comme des amorces pour l'achèvement de seconds brins d'acide nucléique qui sont sensiblement complémentaires desdites matrices d'acide nucléique, ce qui produit ainsi une population de brins codants renfermant des mutations, lesquels brins codants diffèrent les uns des autres par le nombre, la position, et l'identité desdites mutations; et
(d) mettre en contact les produits de l'étape (c) avec une ARN polymérase pour générer une banque d'ARN, ladite banque étant transcrite à partir desdits seconds brins d'acide nucléique.

2. Procédé selon la revendication 1, ledit procédé étant utilisé pour introduire une ou plusieurs mutations dans ladite banque.

3. Procédé servant à réduire la variation de séquence dans une population de molécules d'acide nucléique, ledit procédé comprenant les étapes consistant à:
(a) se procurer une première population de matrices d'acide nucléique monocaténaires de séquence variable, chacune de la quasi-totalité desdites matrices comprenant une séquence codante et une séquence promotrice fonctionnellement liée;
(b) hybrider aux membres de ladite première population une seconde population de fragments d'acide nucléique monocaténaires, lesdits fragments étant de taille plus courte que lesdites matrices d'acide nucléique et lesdits fragments étant sensiblement complémentaires desdites matrices d'acide nucléique, où une sous-population de ladite seconde population comprend une séquence sensiblement identique et s'hybride à une région de séquence variable;
(c) mettre en contact les produits d'hybridation de l'étape (b) à la fois avec une ADN polymérase qui est dépourvue d'activité de déplacement de brin et avec une ADN ligase dans des conditions dans lesquelles lesdits fragments agissent comme des amorces pour l'achèvement de seconds brins d'acide nucléique qui sont sensiblement complémentaires desdites matrices d'acide nucléique; et
(d) mettre en contact les produits de l'étape (c) avec une ARN polymérase pour générer une troisième population de molécules d'ARN, ladite troisième population de molécules d'ARN étant transcrite à partir desdits seconds brins d'acide nucléique et présentant une variation de séquence réduite par rapport à ladite première population de matrices d'acide nucléique monocaténaires.

4. Procédé selon la revendication 3, ledit procédé étant utilisé pour supprimer une ou plusieurs mutations dans ladite première population de matrices d'acide nucléique monocaténaires.

5. Procédé selon la revendication 1 ou 3, dans lequel ledit mélange de fragments d'acide nucléique monocaténaires sensiblement complémentaires est généré par coupure de molécules d'acide nucléique bicaténaires ou par synthèse d'oligonucléotides aléatoires.

6. Procédé selon la revendication 1 ou 3, dans lequel ledit mélange de fragments d'acide nucléique monocaténaires sensiblement complémentaires comprend au moins environ 100 espèces différentes de fragments d'acide nucléique.

7. Procédé selon la revendication 1 ou 3, comprenant en outre une étape consistant à générer des brins complémentaires à partir desdits seconds brins d'acide nucléique, à utiliser lesdits brins complémentaires en tant que ladite population ou ladite première population de matrices d'acide nucléique monocaténaires, et à répéter les étapes (b) et (c) pour un ou plusieurs cycles, puis à répéter l'étape (d).

8. Procédé selon la revendication 1, ledit procédé comprenant en outre une étape consistant à se procurer un ou plusieurs fragments d'acide nucléique monocaténaires qui forment un homoduplex avec lesdites matrices d'acide nucléique monocaténaires et à exécuter l'étape (b) en présence desdits fragments formant un homoduplex.

9. Procédé selon la revendication 1 ou 3, dans lequel ledit promoteur est un promoteur de T7.

10. Procédé selon la revendication 1 ou 3, dans lequel ladite ADN polymérase est l'ADN polymérase de T4.

11. Procédé selon la revendication 1 ou 3, ledit procédé comprenant en outre une étape consistant à amplifier ledit produit de l'étape (c) avant ladite étape de mise en contact (d).

12. Procédé selon la revendication 1 ou 3, comprenant en outre un procédé servant à générer une banque de protéines, ledit procédé comprenant l'étape consistant à:
(e) traduire ladite banque d'ARN pour générer une banque de protéines.

13. Procédé selon la revendication 1 ou 3, comprenant en outre un procédé servant à générer une banque de fusions ARN-protéine, ledit procédé comprenant les étapes consistant à:
(e) relier une molécule acceptrice d'acide aminé à l'extrémité 3'-terminale de ladite séquence codante de chacun de la quasi-totalité des membres de ladite banque d'ARN; et éventuellement
(f) traduire ladite banque d'ARN pour générer une banque de fusions ARN-protéine.

14. Procédé servant à générer une banque d'acides nucléiques, ledit procédé comprenant les étapes consistant à:
(a) se procurer une première population de matrices d'acide nucléique monocaténaires, chacune desdites matrices comprenant une séquence codante;
(b) se procurer une seconde population de molécules d'acide nucléique monocaténaires de séquence variable, ladite première population de matrices d'acide nucléique monocaténaires et ladite seconde population de molécules d'acide nucléique monocaténaires de séquence variable étant sensiblement complémentaires;
(c) hybrider ladite première population de matrices d'acide nucléique monocaténaires à ladite seconde population de molécules d'acide nucléique monocaténaires de séquence variable dans des conditions suffisantes pour former des duplex; et
(d) mettre en contact lesdits duplex avec une ou plusieurs enzymes d'excision/réparation dans des conditions permettant auxdites enzymes de corriger les mésappariements de paires de bases dans lesdits duplex.

15. Procédé selon la revendication 14, ledit procédé comprenant en outre, après l'étape (d), une étape consistant à se procurer une nouvelle population de matrices d'acide nucléique monocaténaires comprenant une séquence codante, lesdites matrices étant obtenues à partir du produit de l'étape (d), à substituer ladite nouvelle population de matrices à ladite première population de matrices, et à répéter les étapes (c) et (d) pour un ou plusieurs cycles.

16. Procédé servant à générer une banque d'acides nucléiques, ledit procédé comprenant les étapes consistant à:
(a) se procurer une population de matrices d'acide nucléique monocaténaires, chacune desdites matrices comprenant une séquence codante;
(b) hybrider à ladite population de matrices d'acide nucléique monocaténaires un mélange de fragments d'acide nucléique monocaténaires sensiblement complémentaires, lesdits fragments étant de taille plus courte que lesdites matrices d'acide nucléique;
(c) mettre en contact chacun des produits d'hybridation de l'étape (b) à la fois avec une ADN polymérase qui est dépourvue d'activité de déplacement de brin et avec une ADN ligase dans des conditions dans lesquelles lesdits fragments agissent comme des amorces pour l'achèvement d'un second brin d'acide nucléique qui est sensiblement complémentaire desdites matrices d'acide nucléique; et
(d) mettre en contact les produits de l'étape (c) avec une ou plusieurs enzymes d'excision/réparation dans des conditions permettant auxdites enzymes de corriger les mésappariements de paires de bases dans lesdits produits.

17. Procédé selon la revendication 16, ledit procédé comprenant en outre les étapes consistant à: se procurer une nouvelle population de matrices d'acide nucléique monocaténaires comprenant une séquence codante et obtenues à partir du produit de l'étape (d), à substituer ladite nouvelle population de matrices à ladite population de matrices d'acide nucléique monocaténaires, et à répéter les étapes (b)-(d) pour un ou plusieurs cycles.

18. Procédé selon la revendication 14 ou 16 dans lequel ladite mise en contact avec lesdites enzymes d'excision/réparation est exécutée *in vitro.*

19. Procédé selon la revendication 14 ou 16 dans lequel ladite mise en contact avec lesdites enzymes d'excision/réparation est exécutée dans une cellule bactérienne.

20. Procédé selon la revendication 1, 3, 14 ou 16 dans lequel lesdites matrices d'acide nucléique monocaténaires sont générées à l'aide d'un phage M13 porteur desdites matrices d'acide nucléique, par digestion d'un brin des matrices d'acide nucléique bicaténaires au moyen de l'exonucléase du gène VI ou de l'exonucléase de lambda, par capture des brins d'acide nucléique individuels biotinylés au moyen de la streptavidine, ou par transcription inverse d'ARN.

21. Procédé selon la revendication 1, 3, 14 ou 16, dans lequel l'étape (b) est exécutée en utilisant entre 1 et environ 1000 molécules d'acide nucléique monocaténaires de séquence variable ou fragments d'acide nucléique monocaténaires pour chacune desdites matrices d'acide nucléique monocaténaires.

22. Procédé selon la revendication 14 ou 16, ledit procédé comprenant en outre l'étape consistant à:
(e) amplifier ledit produit de l'étape (d).

23. Procédé selon la revendication 14 ou 16, dans lequel chacune desdites séquences codantes est fonctionnellement liée à une séquence promotrice.

24. Procédé selon la revendication 23, ledit procédé comprenant en outre l'étape consistant à:
(e) transcrire les produits de l'étape (d) pour générer une banque d'ARN.

25. Procédé selon la revendication 24, comprenant en outre un procédé servant à générer une banque de protéines, lequel procédé comprend l'étape consistant à:
(f) traduire ladite banque d'ARN pour générer une banque de protéines.

26. Procédé selon la revendication 24, comprenant en outre un procédé servant à générer une banque de fusions ARN-protéine, lequel procédé comprend les étapes consistant à:
(f) relier une molécule acceptrice d'acide aminé à l'extrémité 3'-terminale de ladite séquence codante de chacun de la quasi-totalité des membres de ladite banque d'ARN; et éventuellement
(g) traduire ladite banque d'ARN pour générer une banque de fusions ARN-protéine.
